# EUROPEAN PATENT APPLICATION

(11) **EP 4 674 471 A1**
(43) Date of publication of application: **07.01.2026**
(21) Application number: 24763889.3
(22) Date of filing: 27.02.2024
(51) Int. Cl.: A61N 1/36, A61M 25/00

(54) **CATHETER DEVICE AND CATHETER**

(30) Priority: 01.03.2023 JP 2023031346
(71) Applicant: The University of Osaka, Osaka 565-0871 (JP)
(72) Inventor: SASAKURA, Hirozumi, Suita-shi, Osaka 565-0871 (JP); TAMAGAWA, Yuki, Suita-shi, Osaka 565-0871 (JP); TAGAMI, Miki, Suita-shi, Osaka 565-0871 (JP); MASUDA, Hirotada, Suita-shi, Osaka 565-0871 (JP); YAGI, Masakazu, Suita-shi, Osaka 565-0871 (JP); SAWA, Yoshiki, Suita-shi, Osaka 565-0871 (JP); AYUNISA, Nadhifa, Kobe-shi, Hyogo 651-0073 (JP)
(74) Representative: KATZAROV S.A.
(86) International application number: PCT/JP2024/006974
(87) International publication number: WO 2024/181410

(57) **Abstract**

A pleural catheter device (10) includes: a catheter (20) that drains pleural effusion of a subject; an electrode (22) that is provided to the catheter (20) and applies electrical stimulation to a respiratory muscle or a phrenic nerve of the subject; and a controller (32) that performs control of the electrical stimulation by the electrode (22).

## Description

### [Technical Field]

The present invention relates to a catheter device and a catheter.

### [Background Art]

In intensive care units (ICUs), patients are managed using ventilators. Prolonged positive pressure ventilations may deteriorate pulmonary function, and may result in symptoms of atelectasis, for example.

There is a system that contributes to the restoration of negative pressure ventilation achieved by the diaphragm using reaction of the diaphragm to stimuli using electrodes (for example, see Patent Literature (PTL) 1).

### [Citation List]

### [Patent Literature]

[PTL 1] Japanese Patent No. 7153373
[PTL 2] US Patent No. 11052250

### [Summary of Invention]

### [Technical Problem]

The system described in PTL 1 may not be able to restore negative pressure ventilation achieved by the diaphragm. In such cases, the system may not contribute to the improvement in lung function.

The present invention has been conceived to solve the above-described problem, and an object of the present invention is to provide a catheter device and the like that support improvement in lung function.

### [Solution to Problem]

In order to solve the above-described problem, a catheter device according to one aspect of the present invention includes: a catheter that drains pleural effusion of a subject; an electrode that is provided to the catheter and applies electrical stimulation to a respiratory muscle or a phrenic nerve of the subject; and a controller that performs control of the electrical stimulation by the electrode.

Moreover, in order to solve the above-described problem, a catheter device according to one aspect of the present invention includes: a catheter that drains ascites of a subject; an electrode that is provided to the catheter and applies electrical stimulation to a respiratory muscle or a phrenic nerve of the subject; and a controller that performs control of the electrical stimulation by the electrode.

It should be noted that the present invention can be achieved not only as a device, but also as a method including processing components included in the device as steps, a program for causing a computer to execute these steps, a recoding medium such as a computer-readable CD-ROM having the program recorded thereon, or information, data, or signals indicating the program. Moreover, the program, the information, the data, and the signals may be distributed via a communication network such as the Internet.

### [Advantageous Effects of Invention]

The present invention enables the catheter device to support improvement in lung function.

### [Brief Description of Drawings]

[FIG. 1]

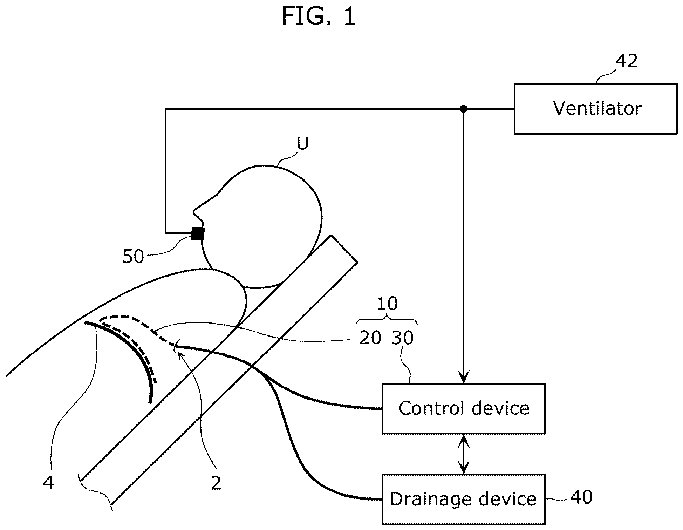

   FIG. 1 is a first explanatory diagram illustrating an example of a configuration of a pleural catheter device according to an embodiment and the pleural catheter device in use.
[FIG. 2]

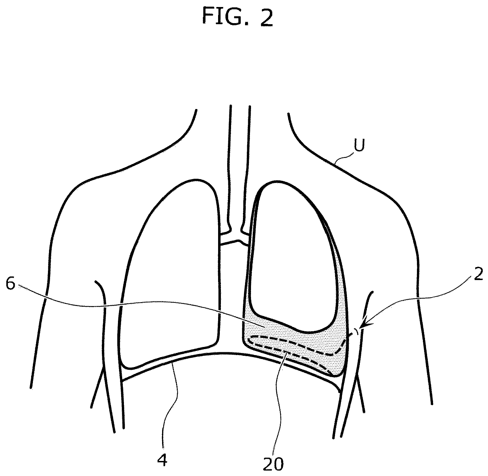

   FIG. 2 is a second explanatory diagram illustrating an example of the configuration of the pleural catheter device according to the embodiment and the pleural catheter device in use.
[FIG. 3A]

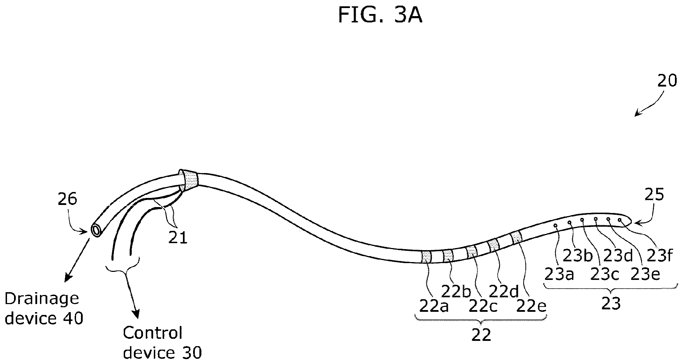

   FIG. 3A is an explanatory diagram schematically illustrating a configuration of a catheter according to the embodiment.
[FIG. 3B]

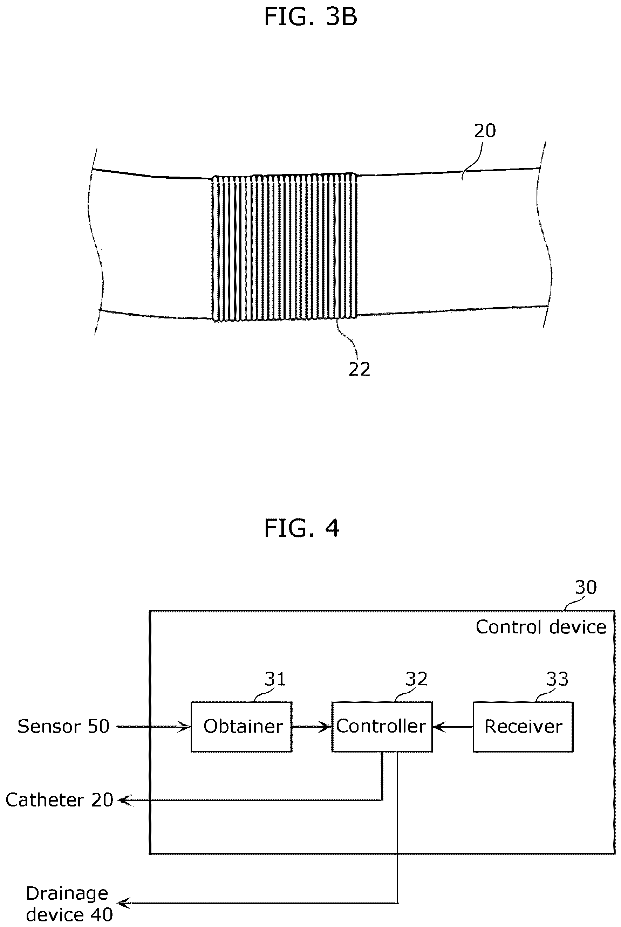

   FIG. 3B is an explanatory diagram schematically illustrating an example of a configuration of an electrode according to the embodiment.
[FIG. 4]
   FIG. 4 is an explanatory diagram illustrating a functional configuration of a control device according to the embodiment.
[FIG. 5]

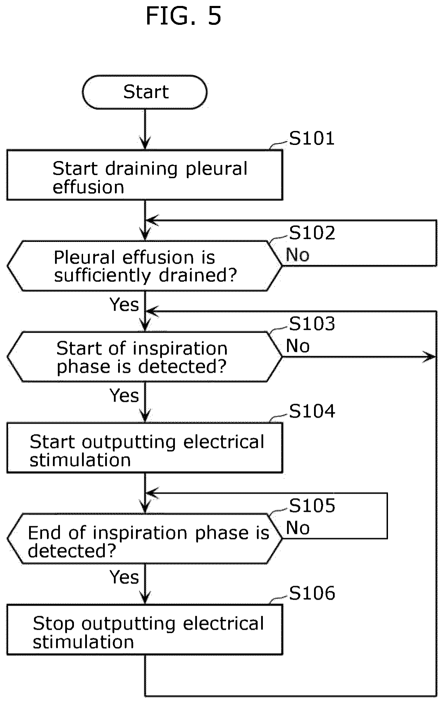

   FIG. 5 is a flowchart illustrating processes of the control device according to the embodiment.
[FIG. 6]

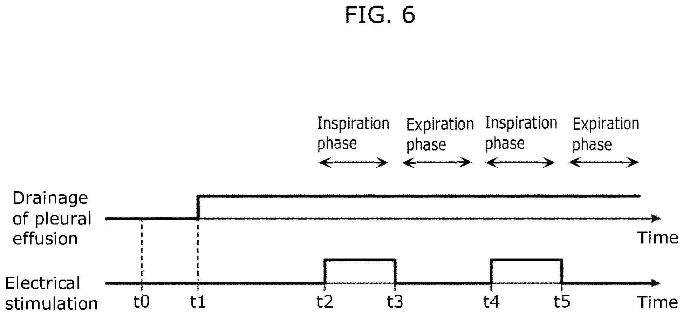

   FIG. 6 is a timing diagram illustrating an example of timings of drainage of pleural effusion and electrical stimulation by the pleural catheter device according to the embodiment.
[FIG. 7]

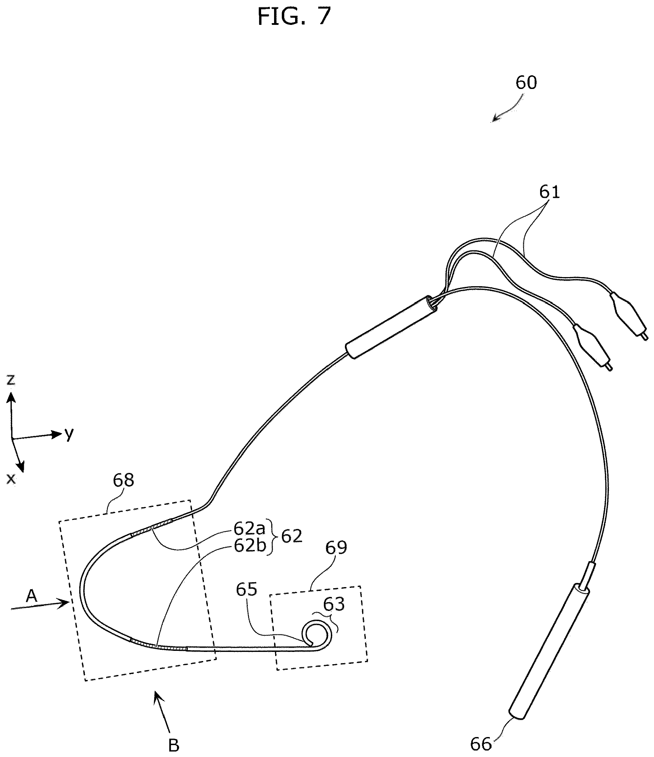

   FIG. 7 is a first explanatory diagram schematically illustrating a configuration of a catheter according to Variation 1 of the embodiment.
[FIG. 8]

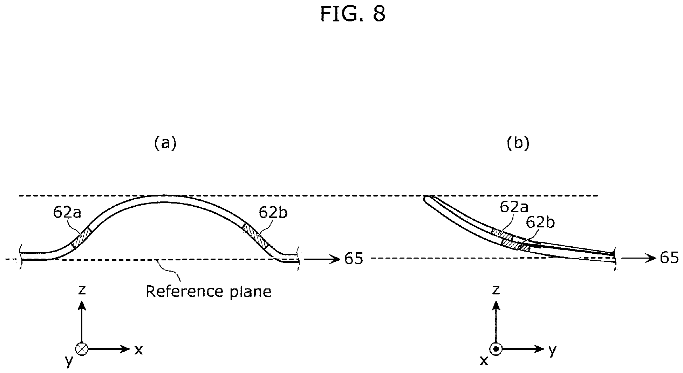

   FIG. 8 is a second explanatory diagram schematically illustrating the configuration of the catheter according to Variation 1 of the embodiment.
[FIG. 9]

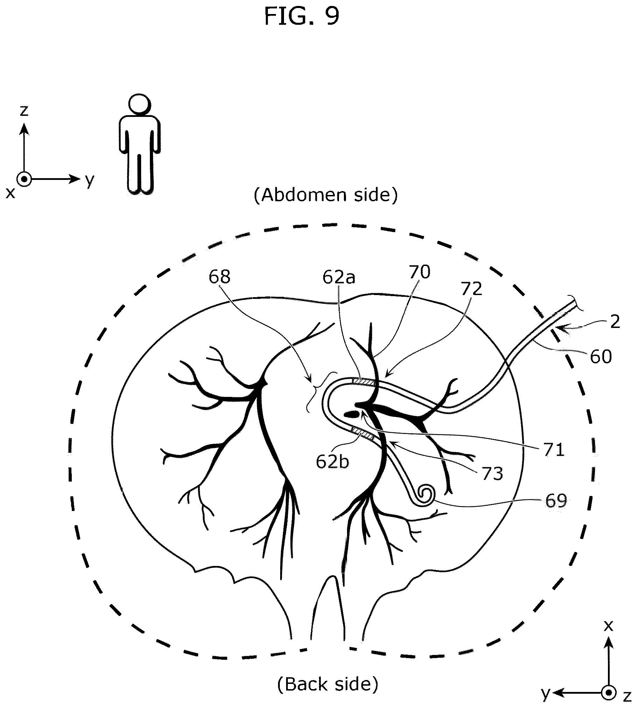

   FIG. 9 is an explanatory diagram schematically illustrating a location where the catheter according to Variation 1 of the embodiment is positioned in the human body.
[FIG. 10]

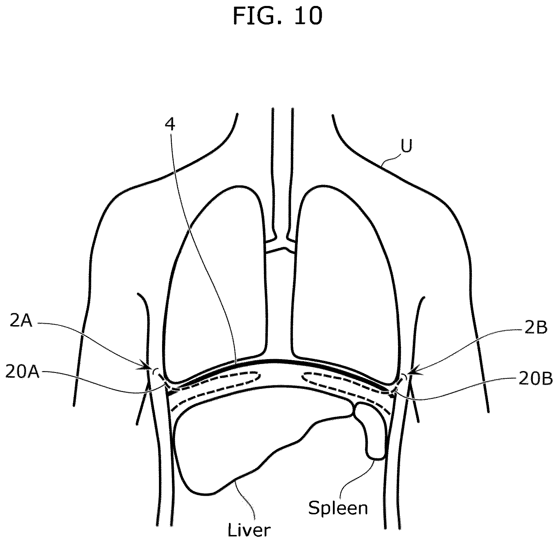

   FIG. 10 is a first explanatory diagram illustrating an example of a peritoneal catheter device according to Variation 2 of the embodiment in use.
[FIG. 11]

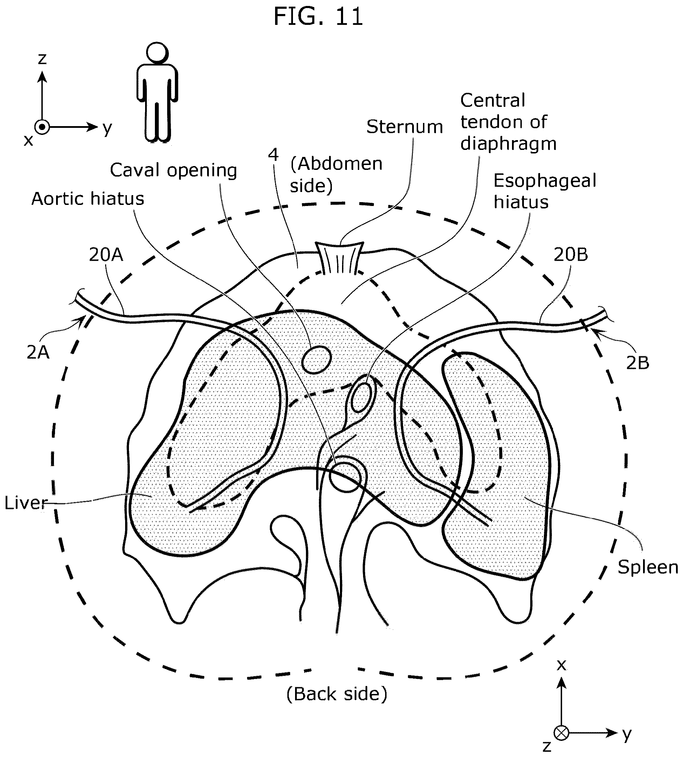

   FIG. 11 is a second explanatory diagram illustrating an example of the peritoneal catheter device according to Variation 2 of the embodiment in use.

### [Description of Embodiments]

### (Underlying Knowledge Forming Basis of the Present Invention)

The inventors have found that the following problems arise in the techniques for symptoms of atelectasis described in the "Background Art" section.

Approximately annually 120,000 patients after cardiac surgery require ventilator management in ICUs due to traffic injuries or acute heart failure.

Usually, when the primary disease improves, the ventilator is removed. However, there are cases where the ventilator is not removed as usual. For example, ventilator-induced diaphragmatic dysfunction (VIDD) may be induced when the strength of the patient's own respiratory muscles decreases during prolonged ventilator support. In addition, pleural effusion may accumulate in the pleural cavity or ascites may accumulate in the peritoneal cavity, due to prolonged bed rest. Moreover, positive pressure ventilation by a ventilator is a nonphysiological ventilation pattern and may not allow sufficient lung distention. These three factors, namely, decreased respiratory muscle strength, accumulation of pleural effusion or ascites, and lung distention failure may overlap. As a result, numerous regions of a lung become unable to distend normally, leading to non-obstructive atelectasis and a decline in the gas exchange function of the lung. This causes a situation where the ventilator cannot be removed, even when the primary disease of the patient is cured. This situation can be a serious problem not only for the patient but also for their family.

Such necessity of continuing intensive care leads to enormous medical costs. For example, it is estimated that annual medical costs of 10 billion yen are incurred, and an enormous amount of human resources is required.

In a super-aged society, the number of patients requiring use of ICUs is increasing. On the other hand, the working population is decreasing, and the resources of healthcare providers are inevitably limited. Therefore, the social significance of solving this issue is very large.

There are two related techniques for stimulating the phrenic nerve with relatively low invasiveness to address VIDD in the ICUs.

The two phrenic nerves that control the activation of the diaphragm run from the brain through the neck, along the left and right sides of the heart through the chest and reach the diaphragm. One of the related techniques is a technique for pacing the diaphragm by stimulating a phrenic nerve in the neck (see PTL 2). The other one of the related techniques is a technique for pacing the diaphragm by stimulating the phrenic nerves from inside the blood vessel by approaching the central veins percutaneously from the left subclavian vein (see PTL 1).

Both techniques are considered to have a certain effect on the improvement in VIDD, but since the pleural effusion is not drained, this prevents the lung distention and does not lead to the resolution of atelectasis. As a result, since the both techniques cannot improve the gas exchange function of a lung, the techniques may not be sufficient to shorten the duration of use of a ventilator.

As described above, there is no effective solution to unmet medical needs of resolving the decreased respiratory function due to decreased respiratory muscle strength or resolving atelectasis. Therefore, cases where the period of receiving ventilator support has been prolonged, and further ventilator-related pneumonia has been developed and, in the worst case, death have continued to occur.

The present invention provides a catheter device that supports improvement in lung function. Specifically, the present invention provides a catheter device that improves symptoms of decreased respiratory function or atelectasis caused by, for example, decreased respiratory muscle strength, accumulation of pleural effusion or ascites, or lung distention failure. The catheter device is a novel device that can electrically stimulate one or more respiratory muscles or a phrenic nerve while draining accumulated pleural effusion or ascites.

The following is an example of an invention obtained from the disclosure in the present specification and description of the effects, etc. obtained from the invention.
(1) A catheter device includes: a catheter that drains pleural effusion of a subject; an electrode that is provided to the catheter and applies electrical stimulation to a respiratory muscle or a phrenic nerve of the subject; and a controller that performs control of the electrical stimulation by the electrode.
   With this aspect, the catheter device performs both (i) drainage of pleural effusion by the catheter that can be placed around the lung of the subject and (ii) electrical stimulation to the respiratory muscle or the phrenic nerve of the subject. The catheter device secures the space around the lung of the subject by draining pleural effusion and also contracts the diaphragm through the electrical stimulation. With this, the catheter device secures the space where the lung can distend and allow the lung to distend in the secured space. This can promote a physiological respiration pattern (i.e., negative pressure ventilation) in the subject. Moreover, the catheter device has an effect of inhibiting muscular atrophy of the diaphragm by moving the diaphragm, which is the largest respiratory muscle, through the electrical stimulation. Moreover, the catheter device achieves the above-described effects with only the catheter placed in the pleural cavity of the subject (in other words, without placing other devices in the body), the physical and mental burden of the subject can be reduced, and contributes to continuously producing the above-described effects. Additionally, the catheter device can reduce the treatment duration and reduce complications or death in mechanically ventilated patients, by fulfilling unmet medical needs of resolving the decreased respiratory function due to decreased respiratory muscle strength or resolving atelectasis. Accordingly, the catheter device supports improvement in lung function.
(2) The catheter device according to (1), in which as the control, after the controller causes the catheter to start draining the pleural effusion, the controller causes the electrode to start the electrical stimulation.
   With this aspect, the electrical stimulation starts after drainage of pleural effusion in the pleural cavity starts, and thus the electrical stimulation can be appropriately performed in a state where the pleural effusion in the pleural cavity is removed and a space where the lung can distend is secured. This can further promote the physiological respiration pattern in the subject. Therefore, the catheter device supports improvement in lung function more appropriately.
(3) The catheter device according to (2), in which the controller obtains timing information indicating a respiratory timing of the subject and output by a sensor that detects the respiratory timing of the subject, and as the control, the controller causes the electrode to repeat outputting the electrical stimulation in synchronization with the respiratory timing indicated by the timing information, while causing the catheter to continuously drain the pleural effusion.
   With this aspect, the catheter device applies the electrical stimulation in synchronization with the respiratory timing of the subject while continuously draining pleural effusion of the subject. With this, the electrical stimulation is applied in synchronization with the respiratory timing while the space around the lung of the subject is secured. This can further promote the physiological respiration pattern through contraction of the diaphragm. Therefore, the catheter device further supports improvement in lung function.
(4) The catheter device according to (3), in which the controller detects an expiration phase and an inspiration phase of the subject based on the timing information, and as the control, the controller: performs control to start outputting the electrical stimulation at a start of the inspiration phase and stop outputting the electrical stimulation at an end of the inspiration phase; and performs control to prohibit outputting the electrical stimulation during the expiration phase.
   With this aspect, the catheter device performs control to output the electrical stimulation during the inspiration phase of the subject, and not to output the electrical stimulation during the expiration phase. With this, when the diaphragm of the subject contracts in the inspiration phase, muscle contraction is excited by the electrical stimulation. This can enhance the effects of enhancing the muscle strength of the diaphragm or inhibiting muscular atrophy. Therefore, the catheter device further supports improvement in lung function.
(5) The catheter device according to any one of (1) to (4), in which the catheter device is connected to a proximal end of the catheter and connected to a drainage device that suctions the pleural effusion via the catheter, and the controller further causes the drainage device to suction the pleural effusion via the catheter.
   With this aspect, the catheter device more easily drains pleural effusion in the pleural cavity through suction of the pleural effusion by the drainage device. Therefore, the catheter device supports improvement in lung function more easily.
(6) The catheter device according to (5), further comprising: a receiver that receives an operation by an operator, in which the controller: controls drainage of the pleural effusion by the catheter in response to reception of an operation related to the drainage of the pleural effusion by the receiver; and controls the electrical stimulation by the electrode in response to reception of an operation related to the electrical stimulation by the receiver.
   With this aspect, since the catheter device controls drainage of pleural effusion and electrical stimulation in response to an operation by the operator, the drainage of pleural effusion and the electrical stimulation can be controlled based on determination by the operator. This can further promote the physiological respiration pattern through contraction of the diaphragm. Moreover, when the operator recognizes that the subject is in an inappropriate state for drainage of pleural effusion or electrical stimulation by the catheter device, control of drainage of the pleural effusion or the electrical stimulation can be inhibited. Consequently, the safety of the drainage of pleural effusion and the electrical stimulation can be improved. Therefore, the catheter device further supports improvement in lung function in a more appropriate state.
(7) The catheter device according to any one of (1) to (6), in which the catheter includes: a hole at a distal end of the catheter; and a side hole located at a position closer to the distal end of the catheter than the electrode is.
   With this aspect, the catheter suctions pleural effusion through both the hole located at the distal end and the side hole located at the position closer to the distal end. If the opening of the catheter is only the hole located at the distal end, suction of pleural effusion may be hindered when the hole is blocked by blood or the like of the subject. Since the catheter further includes a side hole, the catheter can maintain suction of pleural effusion via the side hole even when the opening located at the distal end is blocked. Therefore, the catheter device supports improvement in lung function in a more appropriate state.
(8) The catheter device according to any one of (1) to (7), in which the catheter is inserted into a body of the subject, and a distal end of the catheter is positioned in a pleural cavity of the subject and the electrode is in proximity to the respiratory muscle or the phrenic nerve of the subject.
   With this aspect, the catheter is inserted into the body of the subject, the distal end of the catheter is positioned in the pleural cavity of the subject, and the electrode is in proximity to the respiratory muscle or the phrenic nerve of the subject. With this, the drainage of pleural effusion or the electrical stimulation to the respiratory muscle or the phrenic nerve can be performed more appropriately. Therefore, the catheter device supports improvement in lung function in a more appropriate state.
(9) The catheter device according to (1), in which at least a portion of the catheter includes a curved portion having a curved shape.
   This aspect achieves an effect of enabling further easier insertion of the catheter between the diaphragm and the lung. Moreover, the electrode is more easily in proximity to the respiratory muscle or the phrenic nerve, and the electrical stimulation can be applied to the respiratory muscle or the phrenic nerve more appropriately. Therefore, the catheter device supports improvement in lung function more appropriately.
(10) The catheter device according to (9), in which the curved portion is raised relative to a non-curved portion of the catheter excluding the curved portion.
   This aspect achieves an effect of enabling even easier insertion of the catheter between the diaphragm and the lung, because the shape of the catheter corresponds to the shape of the space between the diaphragm and the lung. Moreover, the electrode is even more easily in proximity to the respiratory muscle or the phrenic nerve, and the electrical stimulation can be applied to the respiratory muscle or the phrenic nerve more appropriately. Therefore, the catheter device supports improvement in lung function more appropriately.
(11) The catheter device according to (9), in which the curved portion includes the electrode.
   With this aspect, the electrode disposed at the curved portion can apply the electrical stimulation to the respiratory muscle or the phrenic nerve more appropriately. Therefore, the catheter device supports improvement in lung function more appropriately.
(12) The catheter device according to any one of (1) to (11), in which the catheter device improves a symptom of decreased respiratory function or atelectasis of the subject through the control by the controller.
   With this aspect, the catheter device supports improvement in the symptom of atelectasis of the subject by controlling the drainage of pleural effusion and the electrical stimulation to the respiratory muscle or the phrenic nerve.
(13) A catheter that drains pleural effusion of a subject, the catheter includes: an electrode that is provided to the catheter and applies electrical stimulation to a respiratory muscle or a phrenic nerve of the subject.
   With this aspect, the catheter is placed around the lung of the subject and performs both drainage of pleural effusion and electrical stimulation to the respiratory muscle or the phrenic nerve of the subject. The catheter secures the space around the lung by draining pleural effusion and contracts the diaphragm through the electrical stimulation to allow the lung to distend in the secured space. This can promote the physiological respiration pattern (i.e., negative pressure ventilation) in the subject. Moreover, the catheter has an effect of inhibiting muscular atrophy of the diaphragm by moving the diaphragm, which is the largest respiratory muscle, through the electrical stimulation. Moreover, since the catheter achieves the above-described effects without placing other devices in the body, the catheter contributes to reduction in physical and mental burdens of the subject and continuous production of the above-described effects. Additionally, the catheter device can reduce the treatment duration and reduce complications or death in mechanically ventilated patients, by fulfilling unmet medical needs of resolving the decreased respiratory function due to decreased respiratory muscle strength or resolving atelectasis. As described above, the catheter supports improvement in lung function.
(14) A catheter device includes: a catheter that drains ascites of a subject; an electrode that is provided to the catheter and applies electrical stimulation to a respiratory muscle or a phrenic nerve of the subject; and a controller that performs control of the electrical stimulation by the electrode.
   With this aspect, the catheter device performs both drainage of ascites by the catheter that can be placed in the peritoneal cavity of the subject and electrical stimulation to the respiratory muscle or the phrenic nerve of the subject. The catheter device inhibits reduction in the space of the lung of the subject that is caused by the diaphragm pushed upward due to ascites accumulation, and contracts the diaphragm by the electrical stimulation. With this, the catheter secures the space where the lung can distend and allows the lung to distend in the secured space to promote the physiological respiration pattern (i.e., negative pressure ventilation) in the subject. Moreover, the catheter device has an effect of inhibiting muscular atrophy of the diaphragm by moving the diaphragm, which is the largest respiratory muscle, through electrical stimulation. Moreover, since the catheter device achieves the above-described effects only with the catheter placed in the peritoneal cavity (i.e., without placing other devices in the body) of the subject, the catheter contributes to reduction in physical and mental burdens of the subject and continuous production of the above-described effects. The catheter device can reduce the treatment duration and reduce complications or death in mechanically ventilated patients, by fulfilling unmet medical needs of resolving the decreased respiratory function due to decreased respiratory muscle strength or resolving atelectasis. As described above, the catheter device supports improvement in lung function.
(15) A catheter that drains ascites of a subject, the catheter includes: an electrode that is provided to the catheter and applies electrical stimulation to a respiratory muscle or a phrenic nerve of the subject.
   With the above-described aspect, the catheter produces the same effects as the effects of the above-described catheter device.
(16) A control method executed by a catheter device, the catheter device including: a catheter that drains pleural effusion of a subject; an electrode that is provided to the catheter and applies electrical stimulation to a respiratory muscle or a phrenic nerve of the subject; and a controller. The control method includes: causing, by the controller, the catheter to start draining the pleural effusion; and after causing the catheter to start draining the pleural effusion by the controller, causing, by the controller, the electrode to start the electrical stimulation.
   The above-described aspect produces the same effects as the effects of the above-described catheter device.
(17) A control method executed by a catheter device, the catheter device including: a catheter that drains ascites of a subject; an electrode that is provided to the catheter and applies electrical stimulation to a respiratory muscle or a phrenic nerve of the subject; and a controller. The control method includes: causing, by the controller, the catheter to start draining the ascites; and after causing the catheter to start draining the ascites by the controller, causing, by the controller, the electrode to start the electrical stimulation.

The above-described aspect produces the same effects as the effects of the above-described catheter device.

Hereinafter, one or more embodiments will be specifically described, with reference to the drawings.

Each of the one or more embodiments described below shows a specific preferred example of the present invention. The numerical values, shapes, materials, structural elements, arrangement and connection of the structural elements, steps, processing order of the steps, etc. mentioned in the following one or more embodiments are mere examples and not intended to limit the present invention. Moreover, among the structural elements in the following exemplary embodiments, structural elements not recited in any one of the independent claims defining the most generic concept of the present invention are described as arbitrary structural elements included in more preferred embodiments. Note that the same structural elements share the same reference signs, and the description thereof may be omitted.

### [Embodiment]

The present embodiment describes a catheter device that supports improvement in lung function. The catheter device according to the present embodiment is a pleural catheter device placed around a lung of a subject. The pleural catheter device can support improvement in lung function using at least drainage of pleural effusion by the catheter.

FIG. 1 and FIG. 2 are schematic diagrams each illustrating an example of a configuration of pleural catheter device 10 according to the present embodiment and the pleural catheter device in use.

Pleural catheter device 10 controls the electrical stimulation of the phrenic nerve of user U (also called the subject) while draining the pleural effusion of user U. User U is, for example, a patient who has symptoms of atelectasis. User U is assumed to be receiving positive pressure ventilation using a ventilator, but this should not be construed as limiting. User U has pleural effusion accumulated in pleural cavity 6 (see FIG. 2).

As illustrated in FIG. 1, pleural catheter device 10 includes catheter 20 and control device 30. Pleural catheter device 10 is connected to drainage device 40 and sensor 50. Note that pleural catheter device 10 may also be connected to ventilator 42.

Note that pleural catheter device 10, drainage device 40, and sensor 50 may be configured as one system. Moreover, pleural catheter device 10, drainage device 40, ventilator 42, and sensor 50 may be configured as one system.

Catheter 20 is a catheter tube connected to control device 30. The inner diameter of catheter 20 is, for example, from approximately 2 mm to 6.5 mm, and the outer diameter is, for example, from approximately 3 mm to 7 mm. The length of catheter 20 is, for example, from approximately 50 cm to 80 cm. The material of catheter 20 is a biocompatible material, and is, for example, silicone or polyurethane.

Catheter 20 is inserted percutaneously into the body of user U (more specifically in pleural cavity 6). On end (also called the proximal end) of catheter 20 is connected to control device 30. Moreover, the other end (also called the distal end) of catheter 20 is inserted into pleural cavity 6 and placed in pleural cavity 6. Catheter 20 includes an electrode (electrode 22, which will be described later).

Catheter 20 is inserted, for example, from the left or right intercostal space of user U into pleural cavity 6 of user U. Specifically, catheter 20 is inserted from opening 2 formed in the body surface of user U into pleural cavity 6 of user U. The distal end of catheter 20 is positioned in pleural cavity 6 of user U, and the electrode is positioned at a position in proximity to one or more respiratory muscles or a phrenic nerve of user U. The position in proximity to the one or more respiratory muscles or the phrenic nerve of user U is, for example, a position on diaphragm 4 of user U. It can be also said that catheter 20 is placed on diaphragm 4 of user U.

Moreover, catheter 20 can also be inserted or positioned surgically into pleural cavity 6 of user U. In this case, for example, catheter 20 is positioned such that catheter 20 exits the body at the epigastrium of user U. However, the position at which catheter 20 exits the body of user U should not be limited to this position.

When catheter 20 is placed in pleural cavity 6 of user U, the electrode can provide electrical stimulation to the one or more respiratory muscle or the phrenic nerve of user U. Moreover, catheter 20 can drain pleural effusion accumulated in pleural cavity 6 of user U through suction by drainage device 40.

Control device 30 controls drainage of pleural effusion by catheter 20, and electrical stimulation by the electrode included in catheter 20. Control device 30 may be implemented by executing a predetermined program by a general-purpose computer or microcontroller, or may be implemented by dedicated hardware.

Drainage device 40 is connected to the proximal end of catheter 20 and drains pleural effusion through catheter 20. Drainage device 40 applies a predetermined suction pressure to the proximal end of catheter 20 when the distal end of catheter 20 is positioned in pleural cavity 6 of user U to suction and drain pleural effusion accumulated in pleural cavity 6 via catheter 20. Drainage device 40 is connected to control device 30 and can perform a suction operation according to a signal received from control device 30. Note that, drainage device 40 can also perform the suction operation according to a signal received from an independent control device, without being connected to control device 30.

Note that drainage device 40 does not have to be a device dedicated to pleural catheter device 10, and may be a general drainage device 40.

Ventilator 42 is a device that performs respiratory management by controlling inspiration and expiration of user U. Ventilator 42 may specifically be a positive-pressure mechanical ventilator. Note that ventilator 42 does not have to be a device dedicated to pleural catheter device 10, and may be a general ventilator 42.

Sensor 50 senses respiration of user U. More specifically, sensor 50 senses inspiration of user U by detecting change in physical quantities such as the expiratory and inspiratory airflow, temperature, carbon dioxide concentration, air pressure, and humidity. Moreover, sensor 50 detects a respiratory timing of user U and obtains timing information indicating the respiratory timing of user U. Sensor 50 provides the timing information to control device 30. Note that when ventilator 42 being used by user U has a function of providing the timing information indicating the respiratory timing, sensor 50 may be such a function of ventilator 42.

Note that in FIG. 2, although the case where pleural effusion has accumulated in pleural cavity 6 on the left side of the chest of user U has been described as an example, there may be cases where pleural effusion has accumulated in the pleural cavity on the right side of the chest of user U. In such cases, catheter 20 is placed in the pleural cavity in which pleural effusion has accumulated (i.e., the pleural cavity on the right side of the chest of user U).

The configuration of catheter 20 will be described in greater detail.

FIG. 3A is an explanatory diagram schematically illustrating the configuration of catheter 20 according to the present embodiment. FIG. 3B is an explanatory diagram schematically illustrating the configuration of electrode 22 according to the present embodiment. The configuration of catheter 20 will be described with reference to FIG. 3A and FIG. 3B.

As illustrated in FIG. 3A, catheter 20 includes electric wire 21, electrode 22, and side hole 23. Of the two end portions of catheter 20, end portion 25 corresponds to the distal end, and end portion 26 corresponds to the proximal end.

Electric wire 21 electrically connects control device 30 and electrode 22, and transmits an electrical signal generated by control device 30 to electrode 22.

Electrode 22 applies electrical stimulation to the one or more respiratory muscles or the phrenic nerve of user U. When catheter 20 is placed in pleural cavity 6 of user U (specifically, placed on diaphragm 4), electrode 22 applies electrical stimulation to the one or more respiratory muscles or the phrenic nerve of user U.

Electrode 22 is disposed on the outside surface of catheter 20 (more specifically, the side wall of catheter 20). Moreover, electrode 22 is disposed at a position a predetermined length away from end portion 25. This is because it is appropriate to dispose electrode 22 in the vicinity of the one or more respiratory muscles or the phrenic nerve, each of which is a target of electrical stimulation, and it is appropriate to position end portion 25 at a position where pleural effusion has accumulated in pleural cavity 6. The predetermined length may be set to approximately correspond to the generally known distance in the human body between the locations of the one or more respiratory muscles or the phrenic nerve and a location where pleural effusion has accumulated. For example, the predetermined length may be from approximately 5 cm to 10 cm, but should not be construed as limiting.

The material of electrode 22 is a biocompatible material, and is, for example, stainless steel, platinum, or iridium.

In greater detail, electrode 22 includes one or more electrodes 22a, 22b, 22c, 22d, and 22e (also called electrode 22a, etc.). Each of electrode 22a, etc. applies electrical stimulation by a change in the electric potential caused by an electrical signal transmitted from control device 30 via electric wire 21. Note that, depending on the position at which each of electrode 22a, etc. is disposed, each of electrode 22a, etc. sometimes cannot apply electrical stimulation effectively to a target position of electrical stimulation. In such cases, electrical stimulation can be effectively applied to a target position of electrical stimulation by changing the selection of electrodes whose electric potential is to be changed among electrode 22a, etc.

Note that, an example in which the number of electrode 22a, etc. is five is described, but the number of electrode 22a, etc. should not be limited to this number. When electrode 22 is one electrode, the above-described electrical stimulation can be applied by changing the electric potential of electrode 22 by using the body of user U as a ground. When electrode 22 corresponds to a plurality of electrodes, the above-described electrical stimulation can be appropriately applied by changing the electric potential between at least two of the plurality of electrodes 22.

Electrode 22 may be, for example, a plate-shaped electrode disposed along the circumference of catheter 20, or an electrode including a conducting wire wound into a coil or a helix around the circumference of the catheter (FIG. 3B, for example).

Side hole 23 is an opening provided in catheter 20 (more specifically, in the side wall of catheter 20). When drainage device 40 applies suction pressure to end portion 26, pleural effusion of user U flows into side hole 23. The pleural effusion that has flowed in passes through the inside of catheter 20 and suctioned by drainage device 40.

Side hole 23 is located at a position closer to end portion 25 than electrode 22 is. Side hole 23 is located in the vicinity of end portion 25 (for example, within approximately 5 cm). In greater detail, side hole 23 includes one or more side holes 23a, 23b, 23c, 23d, 23e, and 23f. Note that, an example in which the number of side hole 23a, etc. is six is described, but the number of side hole 23a, etc. should not be construed as limiting. Moreover, side hole 23a, etc. may be disposed at a variety of positions in the circumference direction.

End portion 25 has an opening. For example, the opening is circular or elliptical, and has a diameter of approximately 1 mm.

End portion 25 is positioned at a position where pleural effusion has accumulated in pleural cavity 6. When drainage device 40 applies suction pressure to end portion 26, pleural effusion of user U flows into end portion 25. The pleural effusion that has flowed in from end portion 25 flows through the inside of catheter 20 and suctioned by drainage device 40. Note that, pleural effusion of user U can also flow into end portion 25 when suction pressure is applied to end portion 26 due to a drop in height.

Moreover, when catheter 20 is inserted into pleural cavity 6 of user U, the opening of end portion 25 also functions as a hole through which a wire that functions as a guide of catheter 20 is inserted.

End portion 26 has an opening. Drainage device 40 is connected to end portion 26. When suction pressure is applied to end portion 26 by drainage device 40, the pleural effusion that has flowed into catheter 20 from the hole of end portion 25 or side hole 23 passes through the inside of catheter 20 and is suctioned by drainage device 40.

As described above, catheter 20 includes, as openings, an opening located at end portion 25 and side hole 23, and pleural effusion of user U flows from these openings. If catheter 20 has only the opening located at end portion 25, suction of pleural effusion may be hindered when end portion 25 is blocked by blood or the like of user U. Since catheter 20 further includes side hole 23 as an opening, catheter 20 can maintain suction of pleural effusion through side hole 23 even when the opening located at end portion 25 is blocked.

The configuration of control device 30 will be described in greater detail.

FIG. 4 is an explanatory diagram illustrating a functional configuration of control device 30 according to the present embodiment.

As illustrated in FIG. 4, control device 30 includes obtainer 31, controller 32, and receiver 33.

Obtainer 31 is connected to sensor 50, and obtains timing information indicating the respiratory timing of user U from sensor 50.

Controller 32 controls electrical stimulation by electrode 22 included in catheter 20. Moreover, controller 32 controls drainage of pleural effusion by catheter 20.

Specifically, controller 32 can cause electrode 22 to apply electrical stimulation while causing catheter 20 to drain pleural effusion.

Moreover, controller 32 can cause electrode 22 to start electrical stimulation after causing catheter 20 to start drainage of pleural effusion. Stated differently, after controller 32 causes electrode 22 to start electrical stimulation, controller 32 can cause catheter 20 to start drainage of pleural effusion.

In the control of drainage of pleural effusion, controller 32 transmits a control signal to drainage device 40 to cause drainage device 40 to suction pleural effusion through catheter 20. Moreover, in the control of electrical stimulation, controller 32 generates an electrical signal for electrical stimulation and transmits the generated electrical signal to electrode 22 via electric wire 21. Any signal pattern may be determined for the electrical signal, and may be, for example, a square wave, a sine wave, an amplitude-modulated wave, a frequency-modulated wave, a burst-modulated wave, or a Gaussian-modulated sine wave. The amplitude of the electrical signal is, for example, from 5 mA to 25 mA. The frequency is, for example, within a range of 20 to 1000 Hz, or may be within a range of 30 Hz to 150 Hz.

When the signal pattern is an amplitude-modulated wave, a frequency-modulated wave, or a burst-modulated wave, and the amplitude or frequency of the electrical signal is gradually raised, thereby alleviating the stimulus to user U immediately after the start of electrical stimulation. For example, when the signal pattern is an amplitude-modulated wave, the amplitude of the electrical signal may be set, for example, within a range of 0.1 mA to 25 mA, electrical stimulation may be started with an electrical signal having an amplitude near the lower limit of the range, and the amplitude may be gradually increased (raised). Moreover, when the signal pattern is a frequency-modulated wave, the frequency of the electrical signal may be set, for example, within a range of 1 Hz to 150 Hz, electrical stimulation may be started with an electrical signal having a frequency near the lower limit of the range, and the frequency may be gradually increased (raised). Moreover, when the signal pattern is a burst-modulated wave, the frequency of the electrical signal may be set, for example, within a range of 20 Hz to 2000 Hz, electrical stimulation may be started with an electrical signal having a frequency near the lower limit of the range, and the frequency may be gradually increased (raised).

Note that, animal testing conducted by the inventors has confirmed that electrical stimulation by an electrical signal having a frequency within the range of 20 Hz to 1000 Hz effectively generates negative pressure in the pleural cavity based on the spontaneous expansion of a lung. Moreover, it has also been confirmed in the same manner that electrical stimulation by an electrical signal having a frequency within the range of 30 Hz to 150Hz generates negative pressure more significantly in the pleural cavity.

Controller 32 may control electrical stimulation according to the respiratory timing of user U while draining pleural effusion continuously. In other words, controller 32 may cause the electrode to repeatedly apply electrical stimulation in synchronization with the respiratory timing of user U indicated by the timing information, while causing catheter 20 to continuously drain pleural effusion.

More specifically, as the control in synchronization with the respiratory timing of user U, controller 32 may detect the expiration phase and the inspiration phase of the respiratory of user U based on the timing information, start electrical stimulation at the start of the inspiration phase, and stop the electrical stimulation at the end of the inspiration phase. In this case, controller 32 prohibits electrical stimulation during the expiration phase.

Controller 32 may control drainage of pleural effusion and electrical stimulation in response to an operation received by receiver 33. In other words, controller 32 may control drainage of pleural effusion in response to the reception of an operation related to drainage of pleural effusion by receiver 33. Moreover, controller 32 may control electrical stimulation by electrode 22 in response to the reception of an operation related to electrical stimulation by receiver 33.

Receiver 33 receives an operation by an operator (for example, a doctor). When receiver 33 receives an operation, receiver 33 provides information indicating the received operation to controller 32. The operation may include, for example, an operation for triggering drainage of pleural effusion, or an operation for triggering electrical stimulation. Moreover, the operation may include an operation for adjusting the drainage amount of pleural effusion, or an operation for adjusting the intensity of electrical stimulation. Receiver 33 may be, for example, a button or a switch that receives an operation by an operator. Moreover, receiver 33 may be an input device such as a touch panel, a touchpad, a keyboard, or a mouse. In this case, receiver 33 may display a guidance image for guiding an operation by an operator on a display screen (not illustrated), and the above-described input device may receive the operation performed by the operator who has seen the guidance image.

The following describes processes of control device 30.

FIG. 5 is a flowchart illustrating the processes of control device 30 according to the present embodiment. The series of processes illustrated in FIG. 5 are processes performed by control device 30 after catheter 20 is inserted into pleural cavity 6 of a subject (i.e., a control method executed by pleural catheter device 10). The series of processes illustrated in FIG. 5 can be also called a treatment method for a subject by using pleural catheter device 10.

Here, an operation is described as an example in which pleural catheter device 10 outputs electrical stimulation in synchronization with the respiratory timing of the subject after draining pleural effusion from the subject. Pleural catheter device 10 supports improvement in lung function of user U with the above operation.

In step S101, controller 32 performs control to start drainage of pleural effusion. Note that drainage of pleural effusion may be started based on an operation by an operator. In other words, controller 32 may start drainage of pleural effusion in response to the reception, by receiver 33, of an operation to start drainage of pleural effusion. In this case, receiver 33 may receive an operation performed by an operator who has seen a guidance image, which is displayed in advance to guide an operation for drainage of pleural effusion.

The drainage of pleural effusion is continuously performed even while the processes in step S102 and subsequent steps are being performed.

In step S102, controller 32 determines whether the pleural effusion in pleural cavity 6 of user U is sufficiently drained as a result of the control in step S101. The determination of whether the pleural effusion in pleural cavity 6 of user U is sufficiently drained can be determined, for example, based on the amount of pleural effusion drained per unit time by suction through drainage device 40. Specifically, when the amount of pleural effusion drained per unit time is less than or equal to a reference value, controller 32 can determine that the pleural effusion in pleural cavity 6 of user U is sufficiently drained. The reference value is, for example, 10 ml per minute. When controller 32 determines that the pleural effusion is sufficiently drained (Yes in step S102), controller 32 proceeds to step S103, and otherwise (No in step S102), controller 32 performs step S102 again. In other words, controller 32 remains in step S102 until the pleural effusion in pleural cavity 6 of user U is sufficiently drained.

In step S103, controller 32 determines whether the start of the inspiration phase of user U is detected. When controller 32 detects the start of the inspiration phase of user U (Yes in step S103), controller 32 proceeds to step S104, and otherwise (No in step S103), controller 32 performs step S103 again. In other words, controller 32 remains in step S103 until controller 32 detects the start of the inspiration phase of user U. Specifically, detection of the start of the inspiration phase of user U includes (i) detecting that an action potential of the phrenic nerve, which can be obtained by electrode 22, has occurred or exceeded a reference value, (ii) detecting that the intrapleural pressure has dropped below the lower limit of the reference range, or (iii) detecting that the inspiratory flow rate, which can be obtained by sensor 50, has increased beyond a reference value.

In step S104, controller 32 starts outputting electrical stimulation by electrode 22. The output of electrical stimulation may be started automatically based on detection of the start of the inspiration phase by controller 32 in step S103, or started based on an operation by an operator. When the electrical stimulation is started based on an operation by an operator, controller 32 may start outputting electrical stimulation in response to the reception, by receiver 33, of an operation to start outputting electrical stimulation. In this case, receiver 33 may receive an operation performed by an operator who has seen a guidance image, which is displayed in advance to guide an operation for starting the output of electrical stimulation.

In step S105, controller 32 determines whether the end of the inspiration phase of user U is detected. When controller 32 detects the end of the inspiration phase of user U (Yes in step S105), controller 32 proceeds to step S106, and otherwise (No in step S105), controller 32 performs step S105 again. In other words, controller 32 remains in step S105 until controller 32 detects the end of the inspiration phase of user U. Specifically, detection of the end of the inspiration phase of user U includes (i) detecting that an action potential of the phrenic nerve, which can be obtained by electrode 22, has become less than or equal to the reference value, (ii) detecting that the intrapleural pressure has exceeded an upper limit of the reference range, or (iii) detecting that the inspiratory flow rate, which can be obtained by sensor 50, has decreased to or below the reference value.

In step S106, controller 32 stops outputting electrical stimulation by electrode 22. The output of electrical stimulation may be stopped automatically based on detection of the end of the inspiration phase by controller 32 in step S105, or stopped based on an operation by an operator. When the electrical stimulation is ended based on an operation by an operator, controller 32 may stop outputting electrical stimulation in response to the reception, by receiver 33, of an operation to stop output of electrical stimulation. In this case, receiver 33 may receive an operation performed by an operator who has seen a guidance image, which is displayed in advance to guide an operation for stopping the output of electrical stimulation. Moreover, the output of electrical stimulation may be automatically ended after a certain period of time (e.g., one second) has elapsed from the start of the inspiration phase, in accordance with the settings set in advance by the operator.

After step S106, the process proceeds to step S103.

With the series of processes illustrated in FIG. 5, pleural catheter device 10 outputs electrical stimulation in synchronization with the respiratory timing of the subject after draining pleural effusion of the subject, thereby supporting improvement in lung function.

Note that the intensity of electrical stimulation can be manually set, and can also be automatically adjusted by controller 32. For example, when the action potential of the phrenic nerve that can be obtained by electrode 22 exceeds the upper limit of the reference range, controller 32 may reduce the intensity of electrical stimulation, and when the action potential of the phrenic nerve falls below the lower limit of the reference range, controller 32 may increase the intensity of electrical stimulation. Moreover, when the intrapleural pressure exceeds the upper limit of the reference range, controller 32 may increase the intensity of electrical stimulation, and when the intrapleural pressure falls below the lower limit of the reference range, controller 32 may reduce the intensity of electrical stimulation. Moreover, when the inspiratory flow rate that can be obtained by sensor 50 exceeds the upper limit of the reference range, controller 32 may reduce the intensity of electrical stimulation, and when the inspiratory flow rate falls below the lower limit of the reference range, controller 32 may increase the intensity of electrical stimulation.

FIG. 6 is a timing diagram illustrating an example of timings of drainage of pleural effusion and electrical stimulation by pleural catheter device 10 according to the present embodiment.

FIG. 6 illustrates the inspiration phase and the expiration phase of user U and timings of drainage of pleural effusion by catheter 20 and electrical stimulation by pleural catheter device 10. The timings of drainage of pleural effusion by catheter 20 and electrical stimulation by pleural catheter device 10 illustrated in FIG. 6 are examples of the timings that can be achieved by performing the series of processes illustrated in FIG. 5 by pleural catheter device 10.

In FIG. 6, at time t0, catheter 20 is inserted percutaneously into pleural cavity 6 of user U. Catheter 20 is inserted by, for example, a doctor.

At time t1, pleural catheter device 10 starts draining pleural effusion by using catheter 20 and drainage device 40 (step S101). After time t1, when the pleural effusion is sufficiently drained, pleural catheter device 10 controls electrical stimulation in synchronization with the respiratory timing of user U.

At time t2, pleural catheter device 10 starts outputting electrical stimulation in response to detection of the start of the inspiration phase of user U (Yes in step S103, step S104).

At time t3, pleural catheter device 10 stops outputting electrical stimulation in response to detection of the end of the inspiration phase of user U (Yes in step S105, step S106). Electrical stimulation will not be output during the expiration phase after time t3 (stated differently, the output of electrical stimulation is prohibited).

After that, pleural catheter device 10 starts outputting electrical stimulation at time t4 in response to detection of the start of the inspiration phase of user U (same as at time t2), and stops outputting electrical stimulation in response to detection of the end of the inspiration phase of user U at time t5 (same as at time t3). Electrical stimulation will not be output during the expiration phase after time t5.

In this way, pleural catheter device 10 outputs electrical stimulation in synchronization with the respiratory timing of user U while draining pleural effusion of user U to move the diaphragm of user U, thereby assisting in spontaneous respiration (i.e., negative pressure ventilation) in user U. Moreover, continuous output of electrical stimulation can prevent degeneration or atrophy of diaphragmatic muscle fibers. Furthermore, continuing drainage of pleural effusion can secure the space where the lung can distend, and avoid obstruction to the lung distention.

### [Variation 1 of Embodiment]

The present variation describes an example different from the above embodiment for the pleural catheter device that supports improvement in lung function.

The pleural catheter device according to the present variation includes a catheter different from the catheter according to the above embodiment. The following describes differences of catheter 60 according to the present variation from catheter 20 (see FIG. 3A) according to the above embodiment. Note that the points that are not particularly described are the same as catheter 20.

FIG. 7 and FIG. 8 are each an explanatory diagram schematically illustrating the configuration of catheter 60 according to the present variation.

FIG. 7 is a perspective view of catheter 60. In FIG. 8, (a) is a schematic diagram illustrating curved portion 68 when viewed in the direction of arrow A in FIG. 7. In FIG. 8, (b) is a schematic diagram illustrating curved portion 68 when viewed in the direction of arrow B in FIG. 7. Description will be given using xyz coordinate axes illustrated in FIG. 7 and FIG. 8.

Catheter 60 includes electric wire 61, electrode 62, and side hole 63. Of the two end portions of catheter 60, end portion 65 corresponds to the distal end, and end portion 66 corresponds to the proximal end. Catheter 60 includes curved portion 68 having a curved shape in a portion of catheter 60. Moreover, a portion including end portion 65 of catheter 60 includes annular portion 69 having an annular shape.

Electric wire 61 electrically connects control device 30 and electrode 62, and transmits an electrical signal generated by control device 30 to electrode 62, as with electric wire 21 of catheter 20.

Electrode 62 (in greater detail, electrodes 62a and 62b) is disposed on the outside surface of catheter 60 (more specifically, the side wall of catheter 60), as with electrode 22 of catheter 20. Although an example in which the number of electrodes 62 is two is illustrated in the figure, the number of electrodes 62 should not be limited to this number.

Side hole 63 is located in the vicinity of end portion 65, as with side hole 23 of catheter 20. Side hole 63 includes one or more side holes (not illustrated) like side hole 23a, etc. in the above embodiment. At least one or more of the one or more side holes are provided in annular portion 69.

End portion 65 and end portion 66 are respectively the same as end portion 25 and end portion 26 of catheter 20.

Curved portion 68 is a portion having a curved shape. Curved portion 68 is at a position from approximately 10 cm to 15 cm away from end portion 65 along catheter 60, for example. Curved portion 68 has a length of from approximately 5 cm to 10 cm along catheter 60, for example. Curved portion 68 has a curved shape as viewed from the z-axis positive direction, as illustrated in FIG. 7. Moreover, curved portion 68 is raised upward (i.e., the z-axis positive direction) from a plane (also called a reference plane) to which a portion of catheter 60 excluding curved portion 68 (also called non-curved portion) belongs (see (a) and (b) in FIG. 8).

Curved portion 68 includes electrode 62. Curved portion 68 is inserted into a phrenic nerve and a lung of user U, and positioned at a position where electrode 62 is in proximity to one or more respiratory muscles or the phrenic nerve of user U. Catheter 60 including curved portion 68 has an effect that catheter 60 can be easily inserted between the diaphragm and the lung, and also has an effect that electrode 62 can easily become in proximity to the one or more respiratory muscles or the phrenic nerve of user U.

Annular portion 69 is a portion having an annular shape and including end portion 65. Specifically, annular portion 69 has a shape that appears annular when viewed from a certain direction (for example, the z-axis positive direction). The annular shape is, for example, a substantially circular shape or a substantially elliptical shape. Note that the annular shape does not have to be a closed shape, and may be a partially open shape.

The width of the annular shape is, for example, from approximately 0.8 cm to 1.5 cm, but should not be construed as limiting. Since annular portion 69 has an annular shape, this is effective to inhibit end portion 65 from damaging tissues in the body of user U when catheter 60 is inserted into the body of user U. Moreover, since annular portion 69 has an annular shape, side hole 63 (specifically, one or more side holes) provided in annular portion 69 can be oriented in various directions. Therefore, it is effective to further inhibit obstruction to the inflow of pleural effusion of user U when drainage device 40 applies suction pressure to end portion 66.

FIG. 9 is an explanatory diagram schematically illustrating a location where catheter 60 according to the present variation is positioned in the body of user U. Description will be given using xyz coordinate axes illustrated in FIG. 9.

FIG. 9 schematically illustrates a cross-sectional view of the body of user U as viewed from above at a position in the vicinity of the lung or the diaphragm (in other words, a cross section parallel to the xy plane and viewed from the positive side of the z direction). In FIG. 9, as a phrenic nerve, nerve trunk 71 and nerves 70 branching from nerve trunk 71 are illustrated.

Curved portion 68 of catheter 60 is positioned to surround nerve trunk 71. This allows electrodes 62 to be positioned at a plurality of positions around nerve trunk 71. Moreover, electrodes 62 are positioned in the vicinity of portion 72 and portion 73 of nerves 70 that are located relatively close to nerve trunk 71. Electrodes 62 positioned at such positions can apply electrical stimulation to nerve trunk 71 and portion 72 and portion 73 of nerves 70 that are located relatively closer to nerve trunk 71. As a result, the effect of the electrical stimulation can be made more pronounced.

Moreover, annular portion 69 of catheter 60 can be positioned at a relatively lower position (i.e., a position in the z-axis negative direction) relative to curved portion 68. When annular portion 69 is positioned at a relatively low position where pleural effusion tends to accumulate, suction of pleural effusion at that position enables more appropriate drainage of pleural effusion.

As described above, catheter 60 can further contribute to support for improvement in the lung function by appropriately drain pleural effusion and making the effect of electrical stimulation more pronounced.

### [Variation 2 of Embodiment]

The present variation describes an example different from the above embodiment for the catheter device that supports improvement in lung function. The catheter device according to the present variation is a peritoneal catheter device placed in the peritoneal cavity of a subject. The peritoneal catheter device can support improvement in lung function using at least drainage of ascites by the catheter.

In general, when ascites accumulates, the diaphragm may be pushed upward and the space in a lung of user U may be reduced, thereby obstructing the respiration of user U. The peritoneal catheter device according to the present variation contracts the diaphragm through electrical stimulation while securing the space where the lung distends by draining ascites to allow the lung to distend in the secured space. This can promote the physiological respiration pattern (i.e., negative pressure ventilation) in the subject.

The peritoneal catheter device according to the present variation is different from the pleural catheter device according to the above embodiment in that the peritoneal catheter device according to the present variation is positioned in the peritoneal cavity, and ascites is drained by the catheter. The following describes differences of the peritoneal catheter device according to the present variation from pleural catheter device 10 according to the above embodiment.

The peritoneal catheter device according to the present variation includes at least a catheter and a control device, as with pleural catheter device 10 according to the above embodiment. The location in the body of user U where the catheter included in the peritoneal catheter device is placed is different from the position of catheter 20 included in pleural catheter device 10. Note that the points that are not particularly described are the same as catheter 20.

FIG. 10 and FIG. 11 are schematic diagrams each illustrating an example of the peritoneal catheter device in use according to the present variation. FIG. 10 schematically illustrates the body of user U as viewed from the front of user U, and FIG. 11 schematically illustrates a cross-sectional view of the body of user U as viewed from below at a position in the vicinity of diaphragm 4, the liver, and the spleen of user U (i.e., a cross-sectional view parallel to the xy plane and viewed from the negative side of the z direction).

The catheter included in the peritoneal catheter device can be placed at least one of a right side of the abdomen of user U or a left side of the abdomen of user U. In FIG. 10 and FIG. 11, an example of the catheter that can be placed on the right side of the abdomen of user U is illustrated as catheter 20A, and an example of the catheter that can be placed on the left side of the abdomen of user U is illustrated as catheter 20B.

As illustrated in FIG. 10 and FIG. 11, catheter 20A is placed between diaphragm 4 and the liver on the upper right side of the abdomen of user U, and exit the body of user U through opening 2A. Moreover, catheter 20B is placed between diaphragm 4, the liver, and the spleen on the upper left side of the abdomen of user U, and exit the body of user U through opening 2B.

The peritoneal catheter device according to the present variation outputs electrical stimulation in synchronization with the respiratory timing of user U while draining ascites of user U to move the diaphragm of user U, thereby assisting in spontaneous respiration (i.e., negative pressure ventilation) in user U. Moreover, continuous output of the electrical stimulation can prevent degeneration or atrophy of diaphragmatic muscle fibers. Furthermore, continuing drainage of ascites can secure the space where the lung can distend, and avoid obstruction to the lung distention.

It should be noted that the present invention can be achieved not only as a device, but also as a method including the processing components included in the device as steps, a program for causing a computer to execute these steps, a recoding medium such as a computer-readable CD-ROM having the program recorded thereon, or information, data, or signals indicating the program. Moreover, the program, the information, the data, and the signals may be distributed via a communication network such as the Internet.

The foregoing has described the catheter device and so on according to the present invention based on the embodiment, but the present invention should not be limited to this embodiment. The scope of the present invention also encompasses embodiments obtained by applying various modifications, which occur to those skilled in the art, to the present embodiment, and embodiments obtained by combining the structural elements and functions in one or more different embodiments as long as these do not depart from the scope of the present invention.

### [Industrial Applicability]

The present invention is applicable to a catheter device that supports improvement in lung function.

### [Reference Signs List]

2, 2A, 2B, opening
4 diaphragm
6 pleural cavity
10 pleural catheter device
20, 20A, 20B, 60 catheter
21, 61 electric wire
22, 22a, 22b, 22c, 22d, 22e, 62, 62a, 62b electrode
23, 23a, 23b, 23c, 23d, 23e, 23f, 63 side hole
25, 26, 65, 66 end portion
30 control device
31 obtainer
32 controller
33 receiver
40 drainage device
42 ventilator
50 sensor
68 curved portion
69 annular portion
70 nerves
71 nerve trunk
72, 73 portion
U user

## Claims

1. A catheter device comprising:
a catheter that drains pleural effusion of a subject;
an electrode that is provided to the catheter and applies electrical stimulation to a respiratory muscle or a phrenic nerve of the subject; and
a controller that performs control of the electrical stimulation by the electrode.

2. The catheter device according to claim 1, wherein
as the control, after the controller causes the catheter to start draining the pleural effusion, the controller causes the electrode to start the electrical stimulation.

3. The catheter device according to claim 2, wherein
the controller obtains timing information indicating a respiratory timing of the subject and output by a sensor that detects the respiratory timing of the subject, and
as the control, the controller causes the electrode to repeat outputting the electrical stimulation in synchronization with the respiratory timing indicated by the timing information, while causing the catheter to continuously drain the pleural effusion.

4. The catheter device according to claim 3, wherein
the controller detects an expiration phase and an inspiration phase of the subject based on the timing information, and
as the control, the controller:
performs control to start outputting the electrical stimulation at a start of the inspiration phase and stop outputting the electrical stimulation at an end of the inspiration phase; and
performs control to prohibit outputting the electrical stimulation during the expiration phase.

5. The catheter device according to any one of claims 1 to 4, wherein
the catheter device is connected to a proximal end of the catheter and connected to a drainage device that suctions the pleural effusion via the catheter, and
the controller further causes the drainage device to suction the pleural effusion via the catheter.

6. The catheter device according to claim 5, further comprising:
a receiver that receives an operation by an operator, wherein
the controller:
controls drainage of the pleural effusion by the catheter in response to reception of an operation related to the drainage of the pleural effusion by the receiver; and
controls the electrical stimulation by the electrode in response to reception of an operation related to the electrical stimulation by the receiver.

7. The catheter device according to claim 1, wherein
the catheter includes:
a hole at a distal end of the catheter; and
a side hole located at a position closer to the distal end of the catheter than the electrode is.

8. The catheter device according to claim 1, wherein
the catheter is inserted into a body of the subject, and
a distal end of the catheter is positioned in a pleural cavity of the subject and the electrode is in proximity to the respiratory muscle or the phrenic nerve of the subject.

9. The catheter device according to claim 1, wherein
at least a portion of the catheter includes a curved portion having a curved shape.

10. The catheter device according to claim 9, wherein
the curved portion is raised relative to a non-curved portion of the catheter excluding the curved portion.

11. The catheter device according to claim 9, wherein
the curved portion includes the electrode.

12. The catheter device according to claim 1, wherein
the catheter device improves a symptom of decreased respiratory function or atelectasis of the subject through the control by the controller.

13. A catheter that drains pleural effusion of a subject, the catheter comprising:
an electrode that is provided to the catheter and applies electrical stimulation to a respiratory muscle or a phrenic nerve of the subject.

14. A catheter device comprising:
a catheter that drains ascites of a subject;
an electrode that is provided to the catheter and applies electrical stimulation to a respiratory muscle or a phrenic nerve of the subject; and
a controller that performs control of the electrical stimulation by the electrode.

15. A catheter that drains ascites of a subject, the catheter comprising:
an electrode that is provided to the catheter and applies electrical stimulation to a respiratory muscle or a phrenic nerve of the subject.
